(19) Europäisches Patentamt
European Patent Office
Office européen des brevets

(11) Veröffentlichungsnummer: **0 037 016**
**B1**

(12) # EUROPÄISCHE PATENTSCHRIFT

(45) Veröffentlichungstag der Patentschrift: **06.04.83**

(51) Int. Cl.³: **C 07 C 149/22,** C 07 C 148/04

(21) Anmeldenummer: **81102033.8**

(22) Anmeldetag: **19.03.81**

(54) **Verfahren zur Extraktion und Gewinnung von Thioglycolsäure aus wässrigen Medien.**

(30) Priorität: **01.04.80 DE 3012580**

(43) Veröffentlichungstag der Anmeldung: **07.10.81 Patentblatt 81/40**

(45) Bekanntmachung des Hinweises auf die Patenterteilung: **06.04.83 Patentblatt 83/14**

(84) Benannte Vertragsstaaten: **BE DE FR GB IT**

(56) Entgegenhaltungen: **US-A-3 980 701**

(73) Patentinhaber: **CASSELLA Aktiengesellschaft, Hanauer Landstrasse 526, D-6000 Frankfurt am Main 61 (DE)**

(72) Erfinder: **Kühlein, Klaus, Dr., Fasanenstrasse 41, D-6233 Kelkheim (DE)**
Erfinder: **Mix, Konrad, Dr., Fuldaerstrasse 24, D-6000 Frankfurt/Main 61 (DE)**

(74) Vertreter: **Urbach, Hans-Georg, Dr., Hanauer Landstrasse 526, D-6000 Frankfurt am Main 61 (DE)**

**EP 0 037 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Extraktion und Gewinnung von Thioglycolsäure aus wässrigen Medien

Die vorliegende Erfindung betrifft ein Verfahren zur Extraktion und Gewinnung von Thioglycolsäure ($HS\text{-}CH_2COOH$) aus wässrigen Medien.

Bei der Synthese fällt Thioglycolsäure neben Nebenprodukten, wie zum Beispiel Kochsalz, in wässrigen Lösungen an und muss hieraus mit Hilfe von organischen Extraktionsmitteln extrahiert werden. Als Extraktionsmittel für diesen Zweck sind zum Beispiel bekannt: Ether, wie beispielsweise Diethylether, Ester, wie beispielsweise Butylactetat, Alkohole, wie beispielsweise n-Butanol, 2-Ethylhexanol. Diese Extraktionsmittel besitzen bei ihrer technischen Anwendung verschiedene Nachteile wie zum Beispiel niedere Siedepunkte, niedere Zündtemperaturen, ungenügende Verteilungskoeffizienz oder hohe Wasserlöslichkeiten. Zur Vermeidung dieser Nachteile ist es bekannt, Thioglycolsäure aus wässrigen Medien mit Hilfe von tert. Butylalkohol oder tert. Amylalkohol (US-PS 2 658 074) zu extrahieren. Auch tert. Butylalkohol und tert. Amylalkohol besitzen für die Extraktion von Thioglycolsäure verschiedene Nachteile. So ist beispielsweise tert. Butylalkohol unbeschränkt mit Wasser mischbar, und auch tert. Amylalkohol ist teilweise in Wasser löslich. Wenn auch die Waserlöslichkeit bei der Verwendung von salzhaltigen wässrigen Lösungen zurückgeht, so ist doch die Wasserlöslichkeit dieser tert. Alkohole, verbunden mit den niederen Siedepunkten von ca. 83°C bzw. 103°C, nachteilig und zwar, weil diese Lösungsmittel aus den Extrakten abdestilliert werden müssen, ehe die Destillation der Thioglycolsäure beginnen kann. Das bedeutet neben dem technischen Aufwand für diese Destillation, dass die Wärmebelastung der Thioglycolsäure, bei der diese zu Polythioestern, Dithioglycolid und anderen Produkten kondensiert (vgl. A. Schöberl und G. Wiehler, Angew. Chem. 66 [1954] 237), um diese Zeit verlängert werden muss, wodurch Ausbeuteeinbussen resultieren können. Im übrigen verfügen sie über einen lästigen Eigengeruch.

Zur Extraktion von niederen Carbonsäuren aus wässrigen Lösungen ist auch ein Verfahren bekannt, bei dem als Extraktionsmittel u.a. Tetraalkylharnstoffe verwendet werden (US-PS 3 980 701). Dieses bekannte Verfahren wird nach Ausführungsbeispiel 2 zur Extraktion von Glycolsäure als nicht geeignet bezeichnet und gibt keine Hinweise auf schwefelhaltige Carbonsäuren.

Es wurde nun überraschenderweise gefunden, dass man die Nachteile der bisherigen Extraktions- und Gwinnungsverfahren für Thioglycolsäure aus wässrigen Medien vermeiden kann, wenn man als Extaktionsmittel einen Tetra-alkylharnstoff der allgemeinen Formel

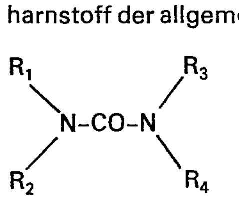

(I)

verwendet. Darin stehen $R_1$, $R_2$, $R_3$ und $R_4$ für Alkylreste mit 1 bis 9 C-Atomen, wobei deren Gesamtzahl mindestens 12 und höchstens 24, vorzugsweise 14 bis 20, betragen soll. Besonders bevorzugt werden als Extraktionsmittel der Tetrabutyl- und Tetrapentylharnstoffe verwendet, wobei wegen der technischen Verfügbarkeit der Tetra-n-butylharnstoff in erster Linie in Frage kommt. Die Verteilungskoeffizienten von Thioglycolsäure, in diesen Tetra-alkylharnstoffen und Wasser sind hoch. So liegt er für Tetra-n-buthylharnstoff bei 4,24. Auserdem sind die genannten Tetra-alkylharnstoffe in Wasser praktisch unlöslich, so dass bei der Extraktion keine Verluste des Extraktionsmittels zu erwarten sind. Die erfindungsgemäss zu verwendenden Extraktionsmittel besitzen relativ hohe Siedepunkte von ca. 250–360°C und sind ausserdem geruchlos.

Die Extraktion und Gewinnung der Thioglycolsäure unter Verwendung der geannten Tetra-alkylharnstoffe wird beispielsweise so durchgeführt, dass eine wässrige ca. 5–30%ige Lösung von Thioglycolsäure, wie sie zum Beispiel nach dem Verfahren von Klason und Bergson (Ber. 39 S. 733) erhalten werden kann, mit einem der erfindungsgemässen Tetra-alkylharnstoffe oder Gemischen solcher Tetra-alkylharnstoffe in an sich bekannter Weise extrahiert wird. Die Lösung der Thioglycolsäure sollte dabei einen pH-Wert zwischen 0 und 4, vorzugsweise zwischen 1 und 3, aufweisen. Die Extraktion selbst kann sowohl diskontinuierlich durch Ausschütteln als auch kontinuierlich zum Beispiel in einem Säulenextraktor nach dem Gegenstromprinzip erfolgen. Bei der Verwendung von Tetra-buthylharnstoff ist es ausreichend, wenn die wässrige Phase zwei- bis dreimal mit demselben Volumen Tetrabutylharnstoff ausgeschüttelt wird. Nach der Extraktion der Thioglycolsäure kann sie durch Destillation unter vermindertem Druck, zweckmässigerweise unter Verwendung einer Kolonne, leicht in reiner Form gewonnen werden. Der vermehrte Druck kann beispielsweise 1 bis 100 mbar betragen.

Beispiel 1

Eine wässrige 15% Thioglycolsäure enthaltende Lösung mit einem pH-Wert von 2,5 wurde zweimal mit demselben Volumen Tetra-n-butylharnstoff ausgeschüttelt. Der in der wässrigen Mutterlauge zurückbleibende Bruchteile der eingesetzten Thioglycolsäure betrug 3 bis 4% und konnte durch ein weiteres Ausschütteln auf unter 1% erniedrigt werden. Die abgetrennten organischen Phasen wurden vereinigt und über einer Vigreux-Kolonne bei 9,3 mbar destilliert. Hierbei ging reine Thioglycolsäure bei einem Siedepunkt von 90°C über.

Beispiel 2

Eine mit Raschigringen gefüllte Kolonne von

ca. 2 m Länge wurde am Kopf mit einer ca. 15%igen wässrigen Thioglycolsäurelösung und am Boden mit Tetra-n-butylharnstoff im Volumenverhältnis 2:1 beschickt. Der am Kopf ablaufende Extrakt enthielt ca. 25% Thioglycolsäure, während die am Boden ablaufende wässrige Mutterlauge praktisch frei von Thioglycolsäure war. Aus dem Extrakt wurde, wie im Beispiel 1 beschrieben, die Thioglycolsäure im Vakuum herausdestilliert. Der Tetra-butylharnstoff wurde ohne Destillation direkt bzw. nach einer Alkaliwäsche zur Entfernung saurer Nebenprodukte der Herstellung, wie zum Beispiel Thiodiglycolsäure oder Dithiodiglycolsäure erneut der Extraktionskolonne zugeführt.

**Patentansprüche**

1. Verfahren zur Extraktion und Gewinnung von Thioglycolsäure aus wässrigen Medien, wobei eine wässrige die Thioglycolsäure enthaltende Lösung mit einem organischen Lösungsmittel extrahiert wird, dadurch gekennzeichnet, dass als Extraktionsmittel ein Tetra-alkylharnstoff der allgemeinen Formel

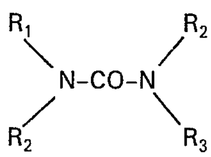

(I)

worin $R_1$, $R_2$, $R_3$ und $R_4$ für Alkylreste mit 1 bis 9 C-Atomen stehen, wobei deren Gesamtzahl mindestens 12 und höchstens 24 betragen soll, verwendet wird.

2. Verfahren nach Anspruch 1, dadurch gekennzeichnet, dass als Extraktionsmittel Tetra-n-butylharnstoff verwendet wird.

3. Verfahren nach den Ansprüchen 1 oder 2, dadurch gekennzeichnet, dass die Extraktion bei einem pH-Wert von 0–4, vorzugswseise 1–3, durchgeführt wird.

4. Verfahren nach den Ansprüchen 1 bis 3, dadurch gekennzeichnet, dass die Thioglycolsäure aus dem Extraktionsmittel durch Destillation unter vermindertem Druck gewonnen wird.

**Claims**

1. Process for the extraction and isolation of thioglycollic acid from aqueous media, wherein an aqueous solution containing the thioglycollic acid is extracted with an organic solvent, characterised in that the extraction agent used is a tetraalkylurea of the general formula

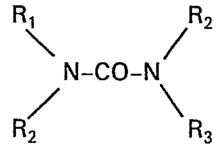

wherein $R_1$, $R_2$, $R_3$ and $R_4$ represent alkyl radicals having 1 to 9 C atoms, the total number of which should be at least 12 and not more than 24.

2. Process according to Claim 1, characterised in that tetra-n-butylurea is used as the extraction agent.

3. Process according to Claim 1 or 2, characterised in that the extraction is carried out at a pH value of 0–4, preferably 1–3.

4. Process according to Claims 1 to 3, characterised in that the thioglycollic acid is isolated from the extraction agent by distillation under reduced pressure.

**Revendications**

1. Procédé pour extraire et isoler l'acide thioglycolique à partir de milieux aqueux, selon lequel on soumet une solution aqueuse contenant l'acide thioglycolique à une extraction par un solvant organique, procécé caractérisé en ce qu'on utilise comme agent d'extraction une tétra-alkylurée répondant à la formule générale I

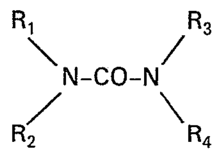

(I)

dans laquelle $R_1$, $R_2$, $R_3$ et $R_4$ représentent des radicaux alkyles contenant chacun de 1 à 9 atomes de carbone et dont le nombre total d'atomes de carbone est d'au moins 12 et d'au plus 24.

2. Procédé selon la revendication 1, caractérisé en ce qu'on utilise la tétra-n-butyl-urée comme agent d'extraction.

3. Procédé selon l'une des revendications 1 et 2, caractérisé en ce qu'on effectue l'extraction à un pH de 0 à 4, de préférence de 1 à 3.

4. Procédé selon l'une quelconque des revendications 1 à 3, caractérisé en ce que l'acide thioglycolique est isolé de l'agent d'extraction par distillation sous pression réduite.